# EUROPEAN PATENT APPLICATION

(11) **EP 1 712 258 A1**
(43) Date of publication of application: **18.10.2006**
(21) Application number: 06007504.1
(22) Date of filing: 10.04.2006
(51) Int. Cl.: A61Q 9/02, A61Q 19/00, A61K 8/73

(54) **Personal cleansing and shaving films**

(30) Priority: 11.04.2005 US 103055
(71) Applicant: National Starch and Chemical Investment Holding Corporation, New Castle, Delaware 19720 (US)
(72) Inventor: Vona, Samuel A. Jr., Somerville New Jersey 08876 (US); Philbin, Michael, T., Hopewell New Jersey 08525 (US)
(74) Representative: Held, Stephan

(57) **Abstract**

Personal care composition in the form of a dry film useful in cleansing or shaving. The film includes at least one water soluble polymer in an amount of about 45 to about 82 weight %, based on total weight of dry film, and, when used for cleansing, at least one surfactant in an amount of about 18 to about 55 weight %; based on total weight of dry film. The at least one water soluble polymer can include blends of polymers. Other additional ingredients for use in the film include plasticizers and/or neutralizing agents. The personal cleansing dissolvable films provide improved aesthetics, foaming, cleansing, moisturizing and lubricity.

## Description

The present invention relates to personal care dissolvable film compositions and to methods of using these dissolvable film compositions in personal care applications. More particularly, the invention relates to dissolvable film compositions containing pullulan, starch and mixtures thereof and their use in cleansing and shaving applications.

Personal cleansing and shaving are huge markets worldwide with a number of different product forms and applications. Product forms include, among others, bars, liquids, gels, foams and powders. Application types include, among others, shampoos, body washes, facial cleansers, hand cleaners, shave foams and makeup removers.

Disadvantages exist with these traditional product forms. For instance, bar soaps can become sloppy after a single use and difficult to travel with, aerosols are restricted on airplanes, and liquid soaps containers can be bulky and leak. Proper use dosage from these product forms can also be difficult to determine without repeated use and experimentation. Often a consumer may use too much of a product, resulting in waste. Finally, the form of the product (*e.g*., solid bar or liquid) can limit the combination of ingredients within a formulation due to ingredient incompatibility in that form.

Use of dissolvable films is known in personal care applications. For instance, dissolvable films have been used to deliver actives for skin treatment. In addition, use of dissolvable films as body cleansers has been mentioned in the art. However, these cleansing films tend to have low surfactant loading and therefore do not function as well as traditional cleansers. Dissolvable films, if they function as well as traditional application forms, can remedy some of the drawbacks associated with those forms, including improving aesthetics, providing compactness, ensuring proper dosing, creating the ability to combine previously incompatible ingredients, and reducing mess.

It has now been discovered that dissolvable films containing pullulan, starch or blends thereof can be used in personal cleansing and shaving applications. These films offer several advantages over conventional cleansing product forms and prior dissolvable film compositions.

The present invention relates to personal cleansing film compositions that function as cleansers or shaving lubricants when dissolved in polar solvent and applied to the skin or hair. The present invention is further related to a method of cleansing skin and hair, as well as a method of shaving using these film compositions. The films contain water soluble polymers (*e.g*., pullulan, starch, or blends thereof). When used for cleansing, the films can also contain one or more surfactants. As shaving films the surfactant ingredient is optional. In one aspect, the invention relates to the use of the personal cleansing dissolvable films with improved aesthetics, foaming, cleansing, moisturization and lubricity.

The following definitions are provided to aid in the description of the present invention:
"Personal cleansing film", as used herein, means a film which is unsupported or supported on a backing, dissolves in polar solvent at room temperature, is applied and distributed on skin and/or hair by a consumer, and removes dirt and/or oil therefrom or provides lubricity and/or hair softening in shaving. The personal cleaning films may be single or multi-layered, embossed, aerated, textured, homogenous or non-homogenous, and/or formed into different shapes and sizes such as, but not limited to, from the size of notebook paper to confetti or to being ground into small flakes of only a few millimeters in length.
"Dissolves in polar solvent", as used herein, means that when the film is added to polar solvent, or polar solvent is added to the film that the film breaks apart or combines with the polar solvent to form a solution or dispersion so as to enable the spread of the composition on skin and/or hair. The wettability or dissolution rates may be modified by one skilled in the art to target a specific delivery profile.
"Plasticizer", as used herein, means any material that will contribute to making a film composition less brittle and more flexible.
"Dry", as used herein, means substantially free of water and other solvent, but does not mean the absence of water or solvent.
"Pullulan", as used herein, means the extracellular bacterial linear polysaccharide of glucose made up of α-1,6-linked maltotriose residues produced from starch by *Aureobasidium pullulans.*
"Natural", as used herein, means derived or partially derived from a plant, animal or bacteria.
"Surfactant", as used herein, means an ingredient that is used in a cosmetic formulation and exhibits the ability to reduce the interfacial tension between two immiscible substances, wets skin and hair surfaces, emulsifies or solubilizes oils, and/or suspends soil and is meant to include amphoteric, anionic, cationic, and nonionic surfactants.
"Polar solvent", as used herein, means a solvent with a dipole moment and is meant to include, but is not limited to, solvents such as water and/or ethanol.
"Aqueous solvent", as used herein, means a solvent containing at least 2 percent water, based on total weight of solvent.
"Non-aqueous solvent", as used herein, means a solvent containing less than 2 percent water, based on total weight of solvent.
"Solvent", as used herein, means any liquid at 25°C that will at least partially dissolve another liquid or a solid.
"Cosmetic", as used herein, means any product intended for use on skin, nail and/or hair.
"Cosmetic ingredient", as used herein, means any ingredient that may be used in cosmetics and/or personal care formulations.
"Pigment", as used herein, means any ingredient that changes the color of a cosmetic formulation.
"Fragrance", as used herein, means any cosmetic ingredient added to a formulation for the purpose of adding, covering, or eliminating an odor.
"Mousse", as used herein, means a personal care product in which the ingredients foam when dispensed from their container without any mechanical action from the user except possibly the shaking of the product in the container prior to actuation of a valve and subsequent dispensing of the internal contents by actuation.
"Shampoo", as used herein, means a cleansing product containing surfactants that is massaged into wet hair, usually thereby creating foam, which is then rinsed from the hair with water, removing at least some soil and/or oils from the hair.
"Bodywash", as used herein, means, a cleaning product distributed over the skin, usually thereby creating foam, which is then rinsed from the body with water removing at least some soil and/or oils from the body.
"Residue", as used herein, means, a material which is exuded from a film upon drying that causes a film to become tacky and block.
"Block" or "Blocking", as used herein, means the condition where films have become tacky and stick or are stuck together.

Accordingly, the present invention provides a personal care composition having at least one water soluble polymer in an amount of about 45 to about 82 weight %; and at least one surfactant in an amount of about 18 to about 55 weight %; wherein the composition is a dry film, and weight % based on total weight of dry film.

The at least one water soluble polymer includes synthetic polymers, natural polymers, or combinations thereof. When the at least one water soluble polymer is one or more natural polymers, those polymers include pullulan, starch, or combinations thereof. The pullulan to starch can be present in the film in a ration of pullulan to starch in the amount of about 100:0 to 75:25.

The personal care film composition can also include at least one plasticizer. When at least starch is used as a water soluble polymer, the plasticizer can be present in an amount greater than 15 percent based upon weight of starch in the composition. In another aspect, the plasticizer can be present in an amount greater than 18 percent based upon weight of starch in the composition.

Suitable surfactants for use in the personal care film composition include sodium lauryl sulfate, ammonium lauryl sulfate, sodium laureth sulfate, ammonium lauryl sulfate, cocamidopropyl betaine, lauramidopropyl betaine, disodium cocoamphodiacetate, sodium lauroyl sarcosinate, TEA-cocoyl glutamate, decyl glucoside or combinations thereof.

The personal care composition can also include at least one neutralizing agent present in an amount sufficient to neutralize the free acid groups of the water soluble polymer.

The personal care film composition can be a cleansing film or a shaving film.

The present invention is also directed towards a method of cleansing skin and/or hair involving wetting the film formed from the personal care composition with water in an amount sufficient to create lather, and applying the lather to the skin and/or hair. The present invention is also directed towards a method of shaving a substrate involving wetting the film formed from the personal care composition with water in an amount sufficient to create lather, and applying the lather to the substrate to be shaved.

In another aspect the present invention is directed towards a method of preventing dissolvable films from blocking. This process involves coating the film formed from the personal care composition with a starch selected from the group consisting of unmodified and modified starch. Such starch can be hydrophobically modified.

The present invention relates to personal cleansing film compositions comprising at least one surfactant and one water soluble polymer, wherein such films function in cleansing hair and/or skin and/or providing lubricity for shaving when dissolved in polar solvent and distributed onto hair and/or skin. Another aspect of the invention is a method of cleansing the skin and/or hair by applying the personal cleansing film to the skin and/or hair. Another aspect of the invention is a method of shaving comprising wetting and applying the personal cleansing film to the hair and/or skin to be shaved prior to bringing the razor in contact with the skin to be shaved. Potential benefits of the invention may include the ability to combine ingredients incompatible in other applications, compactness, single dosing, convenience of use, and small packaging.

In yet another aspect of the invention, the water soluble polymer of the personal cleansing films of the present invention is at least pullulan. Pullulan containing films offer the advantage of remaining flexible (i.e., not so brittle that the film does not form when initially cast or crumbles too easily when handled) with higher surfactant dosages. In general, higher surfactant loading is desirable because a personal cleansing film's effectiveness as a cleanser increases with increasing surfactant concentration in the film.

In yet another aspect of the invention, the water soluble polymer of the personal cleansing films of the present invention includes at least pullulan, one or more surfactants and one or more plasticizers. These pullulan/surfactant/plasticizer containing films offer the advantage of remaining flexible while providing higher surfactant dosages.

In yet another aspect of the invention, the water soluble polymer of the personal cleansing films of the present invention is at least pullulan and starch. These pullulan/starch films offer improved aesthetics such as an increase in the creamy feel and/or lather density of the cleansing film during use.

In another aspect of the invention, the water soluble polymer of the personal cleansing films of the present invention is at least starch, with one or more surfactants present at a dosage above 10 percent by weight of dry film. Such starch based personal cleansing films can also include one or more plasticizers at a dosage greater than 15 percent based on weight of starch in the dry personal cleansing film. These starch/plasticizer personal cleaning films offer the advantage of remaining flexible with higher surfactant loading. Higher surfactant loading is desirable because a personal cleaning film's cleansing effectiveness increases as surfactant concentration increases.

Additional benefits of the films of the invention can include the ability to combine ingredients incompatible with each other in other applications, compactness, single dosing, convenience of use, and small packaging.

The film composition comprises at least one water soluble polymer selected from the group consisting of synthetic polymers, natural polymers, or mixtures thereof.

The water soluble polymer should be present in an amount great enough to effectively form a film that can be handled without crumbling too easily. In one embodiment, the water soluble polymer is present in an amount from about 30 percent to about 44 percent based upon the weight of the dry personal cleansing film. In another embodiment, the water soluble polymer is present in an amount from about 36 percent to about 44 percent based upon the weight of the dry personal cleansing film. In another embodiment, the water soluble polymer is present in an amount from about 36 percent to about 40 percent based upon the weight of the dry personal cleansing film. In another embodiment the water soluble polymer is present in an amount from about 44 percent to about 95 percent based upon the weight of the dry personal cleansing film. In even another embodiment the water soluble polymer is present in an amount from about 30 percent to about 95 percent based on the weight of the dry personal cleansing film.

The following are examples of synthetic water soluble polymers suitable for use in the present invention but in no way is meant to be limiting.

From National Starch and Chemical Company, Bridgewater, New Jersey, AMPHOMER and AMPHOMER LV-71 polymers (octylacrylamide/acrylates/butylaminoethyl methacrylate copolymer), AMPHOMER HC polymer (acrylate/octylacrylamide copolymer), BALANCE 0/55 and BALANCE CR polymers (acrylates copolymer), BALANCE 47 polymer (octylacrylamide/butylaminoethyl methacrylate copolymer), RESYN 28-2930 polymer (vinyl acrylate ('VA')/crotonate/vinyl neodecanoate copolymer), RESYN 28-1310 polymer (VA/crotonate copolymer), FLEXAN polymers (sodium polystyrene sulfonate), DynamX polymer (polyurethane-14 (and) AMP-acrylate copolymer), RESYN XP polymer (acrylate/octylacrylamide copolymer), STRUCTURE 2001 (acrylates/steareth-20 itaconate copolymer) and STRUCTURE 3001 (acrylates/ceteth-20 itaconate copolymer).

From International Specialty Products, Wayne, New Jersey, OMNIREZ-2000 (PVM/MA half ethyl ester copolymer), GANEX P-904 (butylated PVP), GANEX V-216 (PVP/hexadecene copolymer) GANEX V-220 (PVP/eicosene copolymer), GANEX WP-660 (tricontanyl PVP), GANTREZ A-425 (butyl ester of PVM/MA copolymer), GANTREZ AN-119 PVM/MA copolymer, GANTREZ ES 225 (ethyl ester of PVM/MA copolymer), GANTREZ ES-425 (butyl ester of PVM/MA copolymer), GAFFIX VC-713 (vinyl caprolactam/PVP/dimethylaminoethyl methacrylate copolymer), GAFQUAT 755 (polyquaternium-11), GAFQUAT HS-100 (polyquaternium-28) AQUAFLEX XL-30 (Polyimide-1), AQUAFLEX SF-40 (PVP/Vinylcaprolactam/DMAPA Acrylates Copolymer), AQUAFLEX FX-64 (Isobutylene/Ethylmaleimide/Hydroxyethylmaleimide Copolymer), ALLIANZ LT-120 (Acrylates/C1-2 Succinates/Hydroxyacrylates Copolymer), STYLEZE CC-10 (PVP/DMAPA Acrylates Copolymer), STYLEZE 2000 (VP/Acrylates/Lauryl Methacrylate Copolymer), STYLEZE W-20 (Polyquaternium-55), Copolymer Series (PVP/Dimethylaminoethylmethacrylate Copolymer), ADVANTAGE S and ADVANTAGE LCA (Vinylcaprolactam/VP/Dimethylaminoethyl Methacrylate Copolymer), ADVANTAGE PLUS (VA/Butyl Maleate/Isobornyl Acrylate Copolymer); from BASF, ULTRAHOLD STRONG (acrylic acid/ethyl acrylate/t-butyl acrylamide), LUVIMER 100P (t-butyl acrylate/ethyl acrylate/methacrylic acid), LUVIMER 36D (ethyl acrylate/t-butyl acrylate/methacrylic acid), LUVIQUAT HM-552 (polyquaternium-16), LUVIQUAT HOLD (polyquaternium-16), LUVISKOL K30 (PVP), LUVISKOL K90 (PVP), LUVISKOL VA 64 (PVP/VA copolymer) LUVISKOL VA73W (PVP/VA copolymer), LUVISKOL VA, LUVISET PUR (Polyurethane-1), LUVISET Clear (VP/Methacrylamide/Vinyl Imidazole Copolymer), LUVIFLEX SOFT (Acrylates Copolymer), ULTRAHOLD 8 (Acrylates/Acrylamide Copolymer), LUVISKOL Plus (Polyvinylcaprolactam), LUVIFLEX Silk (PEG/PPG-25/25 Dimethicone/Acrylates Copolymer).

From Amerchol, a subsidiary of The Dow Chemical Company, Midland, Michigan, AMERHOLD DR-25 (acrylic acid/methacrylic acid/acrylates/methacrylates).

From Rohm and Haas, Philadelphia, Pennsylvania, ACUDYNE 258 (acrylic acid/methacrylic acid/acrylates/methacrylates/hydroxy ester acrylates.

From Mitsubishi and distributed by Clariant, Muttenz, Switzerland, DIAFORMER Z-301, DIAFORMER Z-SM, and DIAFORMER Z-400 (methacryloyl ethyl betaine/acrylates copolymer), ACUDYNE 180 (Acrylates/Hydroxyesters Acrylates Copolymer), ACUDYNE SCP (Ethylenecarboxyamide/AMPSA/Methacrylates Copolymer), and the ACCULYN rheological modifiers.

From Nalco, Naperville, Illinois, FIXOMER A-30 and FIXOMER N-28 (INCI names: methacrylic acid/sodium acrylamidomethyl propane sulfonate copolymer).

From Noveon, Cleveland, Ohio, a subsidiary of The Lubrizol Corporation, Wickliffe, Ohio, FIXATE G-100 (AMP-Acrylates/Allyl Methacrylate Copolymer), FIXATE PLUS (Polyacrylates-X), CARBOPOL Ultrez 10 (Carbomer), CARBOPOL Ultrez 20 (Acrylates/C10-30 Alkyl Acrylates Copolymer), AVALURE AC series (Acrylates Copolymer), AVALURE UR series (Polyurethane-2, Polyurethane-4, PPG-17/IPDI/DMPA Copolymer); polyethylene glycol; water-soluble acrylics; water-soluble polyesters; polyacrylamides; polyamines; polyquaternary amines; styrene maleic anhydride (SMA) resin; polyethylene amine; and other conventional polymers that are polar solvent soluble or that can be made soluble through neutralization with the appropriate base.

Natural polymers suitable for use as the water soluble polymer in the present invention include any single starch or combination of starches derived from a native source. As used herein a native starch is one as it is found in nature. Also suitable are starches derived from a plant obtained by standard breeding techniques, including crossbreeding, translocation, inversion, transformation or any other method of gene or chromosome engineering to include variations thereof. In addition, starch derived from a plant grown from artificial mutations and variations of the above generic composition, which may be produced by known standard methods of mutation breeding, are also suitable herein.

Typical sources for starches include cereals, tubers, roots, legumes and fruits. Native sources can be corn, pea, potato, sweet potato, banana, barley, wheat, rice, sago, amaranth, tapioca, arrowroot, canna, sorghum, and waxy or high amylose varieties thereof. As used herein, the term "waxy" includes starches containing at least about 95 percent by weight amylopectin, and the term "high amylose" includes starches containing at least about 40 percent by weight amylose or even at least about 70 percent amylose.

Native starches suitable for the present invention can be modified using any modification known in the art, including physical, chemical and/or enzymatic modifications to obtain the desired film attributes.

Physically modified starches, such as sheared starches or thermally-inhibited starches described in the family of patents represented by International Publication No. WO 95/04082, as well as resistant starches described in the family of patents represented by U.S. Patent No. 5,593,503, may be suitable for use herein.

Chemically modified products are also intended to be included as the base material and include, without limitation, those which have been crosslinked, acetylated and organically esterified, hydroxyethylated and hydroxypropylated, phosphorylated and inorganically esterified, cationic, anionic, nonionic, amphoteric and zwitterionic, and succinate and substituted succinate derivatives thereof. Such modifications are known in the art, for example, in Modified Starches: Properties and Uses, Würzburg Ed., CRC Press, Inc., Florida (1986). containing

Conversion products derived from any of the starches, including fluidity or thin-boiling starches prepared by oxidation, enzyme conversion, acid hydrolysis, heat and or acid dextrinization, thermal and or sheared products may also be useful herein.

Further suitable are pregelatinized starches which are known in the art and disclosed, for example, in U.S. Patent Nos. 4,465,702, 5,037,929, 5,131,953, and 5,149,799. Conventional procedures for pregelatinizing starch are also known to those skilled in the art and described for example in Starch: Chemistry and Technology, Vol. III, Industrial Aspects, Chapter XXII, Production and Use of Pregelatinized Starch, R. L. Whistler and E. F. Paschall, Editors, Academic Press, New York (1967).

Any starch or starch blend having suitable properties for use herein may be purified by any method known in the art to remove starch off colors that are native to the polysaccharide or created during processing. Suitable purification processes for treating starches are disclosed in the family of patents represented by European Patent No. 0 554 818 B1. Alkali washing techniques for starches intended for use in either granular or pregelatinized form are also useful and are described in the family of patents represented by U.S. Patent Nos. 4,477,480 and 5,187,272.

Additional suitable starches are those capable of emulsifying or encapsulating an active ingredient so that there is no need for additional encapsulating or emulsifying agents. Such starches include, without limitation, hydroxyalkylated starches such as hydroxypropylated or hydroxyethylated starches, and succinylated starches such as octenyl succinylated or dodecyl succinylated starches. In one embodiment, emulsifying or encapsulating starches are used so that a solution or dispersion of the film material (starch component, active agent, and optional additives) can be stored for later processing. The hydroxyalkylated starches have the added advantage of forming a softer film so that there is less or no need for a plasticizer.

To facilitate processing of the films, the starches can be partially converted to reduce viscosity and allow for production of a high solids starch dispersion/solution, such as a 30% solids starch dispersion/solution. Suitable starches include those with a viscosity of at least about 1000 cps at 10% solids and a viscosity of no more than about 100,000 cps at 30% solids.

The molecular weight of the starch is also important to its functionality in a film, particularly film strength. For example, dextrins are not suitable in the present application unless blended with another water soluble polymer.

The starch component can be a single modified or native starch, a blend of modified starches, or a blend of modified and native starches. Blends can serve in lowering the cost of the film, or in more easily achieving a variety of desirable properties and functionalities.

Examples of commercial starches, with their INCI names, that can be used in the present invention include the following -

From National Starch and Chemical Company, Bridgewater, New Jersey, ULTRASPERSE A (waxy maize starch) polymer, N-LITE LP polymer (hydroxyl propyl starch), AMAZE® polymer (corn starch modified), CELQUAT® LS-50 resin (polyquaternium-4/hydroxypropyl starch copolymer), STRUCTURE® XL polymer (hydroxypropyl starch phosphate), DRY FLO®PC lubricant (aluminum starch octenylsucinate), DRY FLO®AF lubricant (corn starch modified), DRY FLO® ELITE LL lubricant (aluminum starch octenyl succinate (and) lauryl lysine), DRY FLO® ELITE BN lubricant (INCI name: aluminum starch octenyl succinate (and) boron nitride), PURITY®21C starch (zea mays (corn) starch), TAPIOCA PURE (tapioca starch), thermally inhibited corn, potato, tapioca, high amylase, and waxy maize starches sold under the NOVATION trademark, and resistant starches sold under the HI-MAIZE trademark.

From Croda, Cowick Hall, Yorkshire, England, CROSTYLE MFP (trimethyl quaternized maize starch).

From Nalco, Naperville, Illinois, SENSOMER C1-50 (starch hydroxypropyl trimonium chloride).

The natural polymer can also comprise without limitation one or more cellulosic materials such as carboxymethyl cellulose, hydroxypropyl cellulose, microcrystalline cellulose, ethyl cellulose, cellulose acetate phthalate, cationic cellulose derivatives such as polyquaternium-4 (CELQUAT L-200 and CELQUAT H-100 polymers from National Starch and Chemical Company, Bridgewater, New Jersey) and polyquaternium-10 (CELQUAT SC-240C and CELQUAT 230M polymers from National Starch and Chemical Company), or gum, xanthan (such as AMAZE®XT polymer from National Starch and Chemical Company), pullulan, hydrocolloids, carrageenan, alginate, casein, gelatin, and solubilized proteins.

In films containing both synthetic and natural water soluble polymers, the ratio of synthetic to natural water soluble polymer based on the weight of the total water soluble polymer is from about 5:95 to about 95:5; in another embodiment from about 20:80 to about 75:25; in another embodiment from about 25:75 to about 60:40; in another embodiment from about 30:70 to about 55:45; in another embodiment from about 35:65 to about 42:58; in another embodiment from about 29:71 to about 33:67.

In films containing both pullulan and starch polymers, the ratio of pullulan to starch polymer based on the weight of the total water soluble polymer is from about 5:95 to about 95:5; in another embodiment, from about 20:80 to about 75:25; in another embodiment, from about 25:75 to about 60:40; in another embodiment, from about 30:70 to about 55:45; in another embodiment, from about 35:65 to about 42:58; in another embodiment, from about 29:71 to about 33:67; in another embodiment, from about 75:25 to about 99:1; in another embodiment, from about 75:25 to about 85:15.

Personal cleansing films according to the invention contain at least one water soluble polymer or blend of different water soluble polymers. One skilled in the art will recognize that additional materials can be added to the personal cleansing film compositions for modifying the performance or physical properties of the film. For instance, it is known that many synthetic polymers require the addition of a base and/or plasticizer to make the films soluble, less brittle, and/or to optimize aesthetics or performance. Plasticizing agents are also used to improve the flexibility of films containing natural or synthetic polymers. The film should be strong yet flexible, and should not be overly brittle. The film is preferably blocking and moisture resistant so that it does not adhere to itself, yet able to dissolve or disintegrate quickly when exposed to water or other polar solvents, such as when wetted in the hand.

Plasticizing agents are known in the art and include without limitation dimethicone copolyols, polyols, polycarboxylic acids, and polyesters. Examples of useful dimethicone copolyols include, but are not limited to, PEG-12 Dimethicone, PEG/PPG-18/18 Dimethicone, and PPG-12 Dimethicone. Examples of useful polyols include, but are not limited to, ethylene glycol, propylene glycol, sugar alcohols such as sorbitol, manitol, maltitol, lactitol; mono-di- and oligosaccharides such as fructose, glucose, sucrose, maltose, lactose, and high fructose corn syrup solids and ascorbic acid. Examples of polycarboxylic acids include, but are not limited to, citric acid, maleic acid, succinic acid, polyacrylic acid, and polymaleic acid. Examples of polyesters include, but are not limited to, glycerol triacetate, acetylated-monoglyceride, diethyl phthalate, triethyl citrate, tributyl citrate, acetyl triethyl citrate, acetyl tributyl citrate.

Other examples of plasticizers include mineral oils, vegetable oils, triglycerides, lanolins and their derivatives, unsaturated fatty acids and their derivatives, silicones, and some emollients; humectants such as glycerol, sorbitol, lactates (*e.g*., sodium, ammonium and potassium salts), polyols (*e.g*., propylene glycol), polyethylene glycol (200-600), and Sorbeth-30; natural moisturizing factors (NMFs) such as urea, lactic acid, and sodium pyrrolidone carboxylic acid (PCA); liposomes, natural and vegetal moisturizing agents such as glycerol, serine, chitosan PCA, sodium hyaluronate, hyaluronic acid, microsponges, soluble collagen, modified protein, monosodium L-glutamate, lecithins and phospholipids and their derivatives; alpha and beta hydroxy acids such as glycolic acid, lactic acid, citric acid, maleic acid and salicylic acid; polymeric plasticizers such as polysaccharides and their derivatives, polyacrylates, and polyquaterniums; and proteins and amino acids such as glutamic acid, aspartic acid, and lysine.

The plasticizers are preferably present in a plasticizing effective amount. In one embodiment, the plasticizer is present in the personal cleansing film in an amount from about 0 to about 30 percent based on the weight of the dry film composition. In yet another embodiment, the plasticizer is present in an amount from about 5 to about 15 percent based on the weight of the dry film composition. In yet another embodiment, the plasticizer may be present in an amount from about 41 percent to about 70 percent based upon the weight of the dry film; in another embodiment from about 41 to 60 percent based upon the weight of the dry film; in another embodiment from about 41 to about 55 percent based upon the weight of the dry film; in another embodiment from about 45 to 52 percent based upon the weight of the dry film.

In personal cleansing films containing starch in which the starch is at least 60 % (wt/wt) based upon the total weight of the composition, a plasticizer can be present in an amount greater than 15 percent based upon the weight of the starch, but not greater than about 30 percent based upon the weight of the dry film. In another embodiment, the plasticizer can be present in an amount greater than 17 percent based upon the total weight of the starch and less than about 30 percent based upon the weight of the total dry film composition. In even yet another embodiment, the plasticizer can be present in an amount greater than 20 percent based upon the total weight of the starch and less than about 30 percent based upon the weight of the total dry film composition.

Some plasticizers can be added to the solution prior to drying. A portion of the plasticizer (the excess portion) can then be driven off with heat during film formation, resulting in a final product having a higher end dosage. One skilled in the art would know how to adjust the plasticizer to balance film properties.

As known in the art, water soluble polymers containing acidic groups that are insoluble in water are usually used in their neutralized form so as to make them water-soluble or water dispersible. In bi-layer films, it is possible to include the acidic group containing polymer in its unneutralized form in one layer, with a neutralizer in the other layer so that the polymer is neutralized during use.

Suitable neutralizing agents can be included alone or in combination in compositions according to the present invention. These neutralizing agents include alkyl monoamines containing from about 2 to 22 carbon atoms (*e.g*., triethylamine, stearylamine and laurylamine), amino alcohols such as triethanolamine, 2-amino-2-methyl-1,3-propanediol and 2-amino-2-methyl-1-propanol, and inorganic neutralizers such as sodium hydroxide and potassium hydroxide. Other combinations of useful neutralizing agents are described in U.S. Patent No. 4,874,604.

For those polymers requiring neutralization, the neutralizer can be present in an amount effective to neutralize a percentage of the polymer's free acid groups and render the polymer water-soluble or water-dispersible. In one embodiment, the neutralizer is present in an amount sufficient to neutralize the free acid groups of the water soluble polymer, e.g., from about 8 percent to 100 percent neutralization. In another embodiment, the free acid groups of the water soluble polymer can be neutralized from about 25 percent to 100 percent. In another embodiment, the free acid groups of the water soluble polymer can be neutralized from about 50 percent to 100 percent. In another embodiment, the free acid groups of the water soluble polymer can be neutralized from about 70 percent to 100 percent. In yet even another embodiment, the free acid groups of the water soluble polymer can be neutralized from about 80 to 100 percent.

The neutralizer can also be used in excess of 100 percent neutralization to increase the solution pH or to plasticize the resin in addition to neutralization of the polymer acid groups.

The personal cleansing film composition can also contain at least one surfactant known in the art. Surfactants suitable in the present invention include those known in the art for use in personal care compositions, and include nonionic, anionic, cationic, and amphoteric surfactants. Classes of surfactants useful in personal cleansing film compositions of the present invention include the following: alcohols, alkanolamides, alkylaryl sulfonates, alkylaryl sulfonic acids, alkylbenzenes, amine acetates, amine oxides, amines, sulfonated amines and amides, betaines, block polymers, carboxylated alcohol or alkylphenol ethoxylates, diphenyl sulfonate derivatives, ethoxylated alcohols, ethoxylated alkylphenols, ethoxylated amines and/or amides, ethoxylated fatty acids, ethoxylated fatty esters and oils, fatty esters (other than glycol, glycerol, etc.), fluorocarbon-based surfactants, glycerol esters, glycol esters, heterocyclics, imidazolines and imidazoline derivatives, isethionates, lanolin-based derivatives, lecithin and lecithin derivatives, lignin and lignin derivatives, methyl esters, monoglycerides and derivatives, olefin sulfonates, phosphate esters, phosphorous organic derivatives, polymeric (polysaccharides, acrylic acid, acrylamide), propoxylated and ethoxylated fatty acids, propoxylated and ethoxylated fatty alcohols, propoxylated and ethoxylated alkyl phenols, protein-based surfactants, quaternary surfactants, sarcosine derivatives, silicone-based surfactants, soaps, sorbitan derivative, sucrose and glucose esters and derivatives, sulfates and sulfonates of oils and fatty acids, sulfates and sulfonates ethoxylated alkyl phenols, sulfates of alcohols, sulfates of ethoxylated alcohols, sulfates of fatty esters, sulfonates of benzene, cumene, toluene and xylene, sulfonates of condensed naphthalenes, sulfonates of dodecyl and tridecyl benzenes, sulfonates of naphthalene and alkyl naphthalene, sulfonates of petroleum, sulfosuccinamates, sulfosuccinates and derivatives.

Anionic surfactants suitable for use in the personal cleansing film compositions are the alkyl and alkyl ether sulfates. These materials have the respective formulae -

ROSO₃M and RO(C₂H₄O)ₓSO₃M

wherein R is alkyl or alkenyl of from about 8 to about 18 carbon atoms, x is an integer having a value of from 1 to 10, and M is a cation such as ammonium, alkanolamine such as triethanolamine, monovalent metal such as sodium or potassium, and polyvalent metal cation such as magnesium or calcium. The cation M should be selected such that the anionic surfactant component is water soluble. Solubility of the surfactant depends upon the particular anionic surfactants and cations chosen.

In one embodiment, R has from about 8 to about 18 carbon atoms in both the alkyl and alkyl ether sulfates. In another embodiment R has from about 10 to about 16 carbon atoms. In even another embodiment R has from about 12 to about 14 carbon atoms. The alkyl ether sulfates are typically made as condensation products of ethylene oxide and monohydric alcohols having from about 8 to about 24 carbon atoms. The alcohols can be synthetic or they can be derived from fats (e.g., coconut oil, palm kernel oil, tallow). In one embodiment, lauryl alcohol and straight chain alcohols derived from coconut oil or palm kernel oil are preferred. Such alcohols are reacted with between about 0 and about 10, preferably from about 2 to about 5, more preferably about 3, molar proportions of ethylene oxide, and the resulting mixture of molecular species having, for example, an average of 3 moles of ethylene oxide per mole of alcohol, is sulfated and neutralized.

Specific non-limiting examples of alkyl ether sulfates which can be used in the personal cleansing film compositions of the present invention include sodium and ammonium salts of coconut alkyl triethylene glycol ether sulfate, tallow alkyl triethylene glycol ether sulfate, and tallow alkyl hexaoxyethylene sulfate. In one embodiment, alkyl ether sulfates are those comprising a mixture of individual compounds, wherein the compounds in the mixture have an average alkyl chain length of from about 10 to about 16 carbon atoms and an average degree of ethoxylation of from about 1 to about 4 moles of ethylene oxide.

Other suitable anionic surfactants are the water-soluble salts of organic/sulfuric acid reaction products conforming to the formula -

[R¹--SO₃--M]

where R¹ is a straight or branched chain, saturated, aliphatic hydrocarbon radical having from about 8 to about 24, preferably about 10 to about 18, carbon atoms; and M is a cation described hereinbefore. Non limiting examples of such surfactants are the salts of an organic sulfuric acid reaction product of a hydrocarbon of the methane series, including iso-, neo-, and n-paraffins, having from about 8 to about 24 carbon atoms, preferably about 12 to about 18 carbon atoms and a sulfonating agent (*e.g*., SO₃, H₂SO₄) obtained by known sulfonation methods such as bleaching and hydrolysis. Preferred are alkali metal and ammonium sulfonated C₁₀ to C₁₈ n-paraffins.

Still other suitable anionic surfactants are the reaction products of fatty acids esterified with isethionic acid and neutralized with sodium hydroxide where, for example, the fatty acids are derived from coconut oil or palm kernel oil; sodium or potassium salts of fatty acid amides of methyl tauride in which the fatty acids, for example, are derived from coconut oil or palm kernel oil. Other similar anionic surfactants are described in U.S. Patent Nos. 2,486,921, 2,486,922, and 2,396,278.

Succinates are another type of anionic surfactant suitable for use in the personal cleansing film compositions of the present invention. Examples include disodium N-octadecylsulfosuccinnate; disodium lauryl sulfosuccinate; diammonium lauryl sulfosuccinate; tetrasodium N-(1,2-carboxyethyl)-N-octadecyl sulfosuccinate; diamyl ester of sodium sulfosuccinic acid; dihexyl ester of sodium sulfosuccinic acid; and dioctyl esters of sodium sulfosuccinic acid.

Other suitable anionic surfactants include olefin sulfonates having about 10 to about 24 carbon atoms. In this context the term "olefin sulfonates" refers to compounds produced by sulfonation of α-olefins by means of uncomplexed sulfur trioxide, followed by neutralization of the acid reaction mixture in conditions such that any sulfones formed in the reaction are hydrolyzed to give the corresponding hydroxy alkane sulfonates. The sulfur trioxide can be liquid or gaseous, and is usually, but not necessarily, diluted by inert diluents (*e.g*., by liquid SO₂, chlorinated hydrocarbons, etc.) when used in the liquid form, or by air, nitrogen, gaseous SO₂, etc., when used in the gaseous form. Those α-olefins from which the olefin sulfonates are derived are mono-olefins having from about 10 to about 24 carbon atoms, and preferably from about 12 to about 16 carbon atoms. Preferably, they are straight chain olefins. In addition to true alkene sulfonates and a proportion of hydroxy alkane sulfonates, the olefin sulfonates can contain minor amounts of other materials, such as alkene disulfonates, depending upon reaction conditions, proportion of reactants, nature of the starting olefins and impurities in the olefin stock, and side reactions during the sulfonation process. A non-limiting example of such an α-olefin sulfonate mixture is described in U.S. Pat. No. 3,332,880.

Another class of anionic surfactants suitable for use in the personal cleansing film compositions are the β-alkyloxy alkane sulfonates. Examples of suitable anionic surfactants for use in the personal cleansing film compositions include ammonium lauryl sulfate, ammonium laureth sulfate, trimethyl amine lauryl sulfate, trimethyl amine laureth sulfate, triethanolamine lauryl sulfate, triethanolamine laureth sulfate, monoethanol amine lauryl sulfate, monoethanol amine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, arumonium lauroyl sulfate, sodium cocoyl sulfate, sodium lauroyl sulfate, potassium cocoyl sulfate, potassium lauryl sulfate, triethanol amine lauryl sulfate, triethanol amine lauryl sulfate, monoethanol amine cocoyl sulfate, monoethanol amine lauryl sulfate, sodium tridecyl benzene sulfonate, sodium dodecyl benzene sulfonate, and combinations thereof.

Suitable amphoteric or zwitterionic detersive surfactants for use in the personal cleansing film composition herein include those which are known for use in hair care or other personal care cleansing composition, and which contain a group that is anionic at the pH of the personal cleansing film composition. Non-limiting examples of suitable zwitterionic or amphoteric surfactants are described in U.S. Patent Nos. 5,104,646 and 5,106,609.

Amphoteric surfactants suitable for use in the personal cleansing film composition are well known in the art, and include those surfactants broadly described as derivatives of aliphatic secondary and tertiary amines in which the aliphatic radical can be straight or branched chain, wherein at least one of the aliphatic substituents contains from about 8 to about 18 carbon atoms and at least one contains an anionic water solubilizing group such as carboxy, sulfonate, sulfate, phosphate, or phosphonate.

Zwitterionic surfactants suitable for use in the personal cleansing film composition are well known in the art, and include those surfactants broadly described as derivatives of aliphatic quaternary ammonium, phosphonium, and sulfonium compounds in which the aliphatic radicals are straight or branched chain, and wherein at least one of the aliphatic substituents contains from about 8 to about 18 carbon atoms and at least one contains an anionic group such as carboxy, sulfonate, sulfate, phosphate or phosphonate. Zwitterionic surfactants such as betaines are preferred.

Non-limiting examples of other nonionic, anionic, zwitterionic, amphoteric or additional surfactants suitable for use in the personal cleansing film compositions are described in McCutcheon's Emulsifiers and Detergents 1989 Annual, MC Publishing, Glen Rock, New Jersey , as well as U.S. Patent Nos. 3,929,678, 2,658,072, 2,438,091 and 2,528,378.

Surfactants in the present invention can be used alone or in combination. The concentration of surfactant in the personal cleansing film composition can vary depending upon the cleansing or lather performance desired, the surfactant selected, desired product concentration, the presence of other components in the composition, as well as other factors well known in the art. In one embodiment, the concentration of surfactant in the personal cleansing film is from about 10 percent to about 70 percent based upon the dry weight of the film; in another embodiment, from about 20 percent to about 60 percent based upon the weight of the dry film; in another embodiment, from about 30 percent to about 55 percent based upon the weight of the dry film; in another embodiment, from about 10 percent to about 20 percent based upon the weight of the dry film; in another embodiment, from about 10 percent to about 30 percent based upon the weight of the dry film; in another embodiment, from about 10 percent to about 55 percent based upon the weight of the dry film; in another embodiment, from about 20 percent to about 30 percent based upon the weight of the dry film; in another embodiment, from about 20 percent to about 55 percent based upon the weight of the dry film; in another embodiment, from about 20 percent to about 70 percent based upon the weight of the dry film in another embodiment, from about 30 percent to about 60 percent based upon the weight of the dry film; in another embodiment, from about 30 percent to about 70 percent based upon the weight of the dry film; in another embodiment, from about 55 percent to about 60 percent based upon the weight of the dry film; in another embodiment, from about 55 percent to about 70 percent based upon the weight of the dry film; in another embodiment, from about 60 percent to about 70 percent based upon the weight of the dry film; in another embodiment, from about 41 to about 70 percent based upon the weight of the dry film; in another embodiment, from about 41 to about 60 percent based upon the weight of the dry film; in another embodiment, from about 41 to about 55 percent based upon the weight of the dry film.

The personal cleansing film composition can also include other optional film forming and water soluble ingredients known in the art. These optional ingredients include, without limitation, thickeners, emulsifiers, aesthetic modifiers, UV filters, humectants (such as hydroxyethyl urea, available from National Starch and Chemical Company under the trademark HYDROVANCE), lubricants, skin whitening ingredients, silicones, powders, de-viscosifying agents, moisturizers, emollients, solvents, chelating agents, vitamins, antioxidants, botanical extracts, pH adjusting agents, preservatives, fragrances, waterproofing agents, active ingredients (anti-aging agents, firming or toning agents, etc.), dyes, pigments, colors, polymers, conditioning agents, rheology modifiers, surfactants, opacifiers, foaming agents, heat generating agents and/or effervescing agents, glitter and decorative beads and shapes.

The effervescing agents can be one or more materials that effervesce when placed in contact with water. In one embodiment, the effervescent element of the film is comprised of two components. Suitable first components comprise any acids present in dry solid form such as C₂-C₂₀ organic mono- and poly-carboxylic acids. In another embodiment, the first component can be α- and β-hydroxycarboxylic acids; C₂-C₂₀ organosulfur acids such as toluene sulfonic acid; and peroxides such as hydrogen peroxide. In one embodiment hydroxycarboxylic acids include adipic, gutaric, succinic, tartaric, malic, maleic, lactic, and/or salicylic acids, as well as acid forming lactones such as gluconolactone and glucarolactone. In another embodiment, the acid is citric acid. Also suitable as the acid material are water soluble synthetic or natural polymers such as polyacrylates (e.g., encapsulating polyacrylic acid), cellulosic gums, polyurethane and polyoxyalkylene polymers. The term "acid" includes any substance that, when dissolved in deionized water at 1% concentration, will have a pH of less than 7; in another embodiment less than 6.5; in another embodiment less than 5. The acids are in solid form at 25°C in one embodiment (i.e., having melting points no less than 25°C). Acid concentration can range from about 0.5 to about 80 percent based on the final weight of the water soluble film; in another embodiment from about 10 to about 65 percent; in another embodiment from about 20 to 40 percent.

Suitable second components of the effervescent element include alkaline materials. An alkaline material is any substance which can generate a gas (*i.e.,* effervesce) such as carbon dioxide, nitrogen or oxygen when contacted with water and the acidic material of the first component. Suitable alkaline materials include anhydrous salts of carbonates and bicarbonates and alkaline peroxides. In one embodiment, the alkaline material is sodium or potassium bicarbonate. Amounts of alkaline material can range from about 1 to about 40 percent based upon the weight of the water soluble film; in another embodiment from about 5 to 35 percent; in another embodiment from about 15 to about 30 percent; in another embodiment from about 25 to about 35 percent.

The acid and alkaline components of the effervescing element can be physically separated until combined with water. Methods of separation include formulating a bi-layer film, wherein one layer contains the acid component and the other layer contains the alkaline component. Another example of a method of physical separation is encapsulation of at least one component in a third material. Such methods of producing bi-layer films and encapsulation of acid or basic materials are known in the art.

The heat-generating component of a film can be one material or a combination of more than one material that generates heat when placed in contact with water. Examples of heat-generating combinations include combinations of acids and bases. In one embodiment, the heat-generating combination is a combination of an oxidizing reagent and a reducing agent. Such oxidizing and reducing agents may be selected broadly from the various compounds of this nature available. Examples of oxidizing agents include chlorates, perchlorates, permanganates, persulfates, peroxides, nitrates, metal oxides, such as copper oxide, lead oxide, and iron oxide, and perborates. In one embodiment the oxidizing agent includes hydrogen peroxide, urea peroxide, sodium peroxide, sodium perborate, sodium persulfate, ammonium persulfate, potassium persulfate or mixtures thereof. Reducing agents include magnesium, zinc, aluminum and iron; sulfites, thiosulfates, thioureas, imidazolinethiones, thiotrazoles, thiopyridines, thio-pyrimidines, thiols, thio-acids, sulfoxides, xanthates, ortho- and para-polyhydroxy benzenes, aldehydes and glycols.

The oxidizing and reducing agents can be physically separated until combined with water. Such methods of separation include formulating a bi-layer film wherein one layer contains the oxidizing component and the other layer contains the reducing component. Another method of physical separation includes encapsulation of at least one component in a third material.

Single components that generate heat when combined with water are those having an appreciable heat of solution or dilution in water (e.g., the combination of water and ethylene glycol, or the combination of water and salts such as aluminum sulfate, calcium chloride, copper sulfate, ferric chloride, magnesium chloride, magnesium sulfate, etc.). In one embodiment, the single heat-generating component can range from about 1 to about 40 percent based upon the weight of the water soluble film; in another embodiment from about 5 to 35 percent; in another embodiment from about 15 to about 30 percent; in another embodiment from about 25 to about 35 percent.

The personal care cleansing and/or shaving films of the present invention can be formed by techniques known in the industry. For example, the water soluble polymer constituent can be dispersed with other film components in water or solvent and dried into film form. Alternatively, the water soluble polymer and other dry components can be blended together and then dispersed with any additional film components in water and/or solvent and dried into film form. Films can be formed from such dispersions or solutions by shaping them into a solidified form of suitable thickness by techniques known in the art (*e.g.,* wet casting, freeze-drying, and extrusion molding). The dispersion or solution can also be directly coated or sprayed onto another product and dried to form a film.

In one embodiment films according to the present invention are processed by preparing a coating formulation. This coating formulation is prepared by making a solution or dispersion of the film components, applying the mixture to a substrate, using knife, bar or extrusion die coating methods, drying the coated substrate to remove at least part of the solvent, and removing the film from the substrate. Examples of substrates include silicone elastomers, metal foils and metalized polyfoils, composite foils or films containing polytetrafluoroethylene materials or equivalents thereof, polyether block amide copolymers, polyurethane, polyvinylidene, polyester, and other such materials useful in the art as releasable substrates. The personal care cleansing film can be dried at standard temperature and/or pressure, or at lower or elevated temperature and/or pressure compared to standard conditions.

The films of the present invention can be packaged according to the various types and methods as known in the art. For example, they can be packaged in cartridges, packettes, roles, or as tape in a tape dispenser. They can be packaged as individual or one-use doses or together side-by-side in a multi-pack where the films are in contact with each other or are separated by some sort of barrier. The packaging chosen will depend upon the targeted end use application. For instance, in an application where a user's hands are already wet when retrieving a film, packaging the films in separate individually sealed packets is more advantageous than in a multi-pack, thereby keeping moisture away from the other films. Where a user will remove a film with dry hands, films may be packaged together without separate packaging.

Solid particles can also be incorporated in the films. Solid particles include, for example, exfoliating beads, encapsulated ingredients, or decorative elements.

Dissolution rate is determined by measuring the time it takes a square inch of film to disintegrate in a beaker of polar solvent. In one embodiment, the personal cleansing film disintegrates in 25°C water in about 15 minutes or less. In another embodiment, the personal cleansing film disintegrates in 25°C water in about 10 minutes or less. In another embodiment, the personal cleansing film disintegrates in 25°C water in about 5 minutes or less. In another embodiment, the personal cleansing film disintegrates in 25°C water in about 2.5 minutes or less. In another embodiment, the personal cleansing film disintegrates in 25°C water in about 1 minute or less. In another embodiment, the personal cleansing film disintegrates in 25°C water in 45 seconds or less. In another embodiment, the personal cleansing film disintegrates in 25°C water in about 30 seconds or less. In another embodiment, the personal cleansing film dissolves in 25°C ethanol in about 5 minutes or less. In another embodiment, the personal cleansing film disintegrates in 25°C ethanol in about 2.5 minutes or less. In another embodiment, the personal cleansing film disintegrates in 25°C ethanol in about 1 minute or less. In another embodiment, the personal cleansing film disintegrates in 25°C ethanol in about 45 seconds or less. In another embodiment, the personal cleansing film disintegrates in 25°C ethanol in about 30 seconds or less.

The films may not be completely dried in that some degree of water or other solvent remains. The amount of solvent present in the film can be controlled to obtain desired functionality. For example, more solvent typically results in a more flexible film, while too much solvent can result in a film that blocks and is tacky. Some solvent may generally remain in the personal cleansing film as used. In one embodiment, the remaining solvent in the personal cleansing film is in the range of from about 0 to about 25 weight percent, based on the weight of the film; in another embodiment, from about 1 to about 20 weight percent solvent remains; in another embodiment, from about 5 to 16 weight percent solvent remains; in another embodiment, about 10 to 15 weight percent solvent remains; in another embodiment, from about 0.1 to 4 weight percent solvent remains.

Film thickness is determined by the liquid coating thickness. Liquid coating thickness can be modified on a Braive Laboratory Bar Coater by adjusting the GAP setting on the film coater. In one embodiment, the films are cast with GAP settings from 25 to 80; in another embodiment, with a setting of 25 to 60; in another embodiment, with a setting of 25 to 40; in another embodiment, with a setting of 40 to 50; in another embodiment, with a setting of 40 to 60; in another embodiment, with a setting of 50 to 60. Other laboratory coaters may be used to cast films of the present invention, the coating thickness setting being dependent upon the scale of the particular coating used and the desired thickness of the resulting film desired. Appropriate liquid coating thickness in film casting can easily be determined by one with skill in the art.

The thickness of the cleansing film can be in the range of about 1 to 500 microns. In one embodiment the film has a thickness from about 25 to about 100 microns. In another embodiment the film has a thickness from about 25 to 60 microns. In yet another embodiment the film has a thickness from about 25 to about 50 microns.

The resultant films are lightweight and easy to carry. They are sufficiently strong and flexible so as to be easily dispensable and handled.

The films exhibit moisture and blocking resistance, yet are wetted when exposed to water or a polar solvent followed by rapid dissolution and/or disintegration. The speed at which the cleansing films wet and dissolve can be modified by one skilled in the art to target a specific delivery profile. For example, more rapid dissolution of carboxylated water soluble polymers can be achieved using neutralization and/or plasticization. Neutralization of carboxylic groups of water soluble polymers creates charged groups along the polymer backbone wherever a carboxyl group is neutralized. The charged polar groups make these sections of the polymer more soluble in polar solvents than if these carboxyl groups were not neutralized.

The personal cleansing films of the present invention permit the use of polymers and surfactants that are difficult or impossible to be used together in other application forms. The formulation of these polymers and surfactants into a personal cleansing film gives a novel, fun application and overcomes some of the limitations associated with other applications. For example, some anionic and cationic polymers and/or surfactants can be combined and formed into films in ratios that would form an insoluble precipitate in aqueous solutions, would be hazy, or would have unacceptable rheology. As another example, a polymer that forms a gel at high pH can be incorporated into one film layer of a bi-layer film, with an activating base incorporated into the other layer. Once the bi-layer film is dissolved, the two materials commingle and thicken, giving lubricity.

A particularly suitable cleansing film composition of the present invention includes pullulan and at least one surfactant. Another particularly suitable cleansing film composition of the present invention includes pullulan, at least one starch such as hydroxypropylated high amylase corn starch, at least one surfactant and glycerin. Another particularly suitable cleansing film composition of the present invention includes pullulan in an amount of 33 to 66 percent by weight of the film, and at least one surfactant in an amount of 30 to 55 percent by weight of the film. Another particularly suitable cleansing film composition of the present invention comprises 33 to 66 percent pullulan, 30 to 55% surfactant, 10 to 37 percent modified starch. Another particularly suitable cleansing film composition of the present invention comprises 33 to 66 percent by weight pullulan, 30 to 55 percent by weight surfactant, 10 to 37 percent by weight modified starch and 0.1 to 5 percent by weight glycerin.

A user of the personal care composition can apply the film to the hair or skin in a number of different ways. One method of application comprises wetting the hands, placing the composition in the hands, distributing the film over the hands and then applying the wetted film by passing the hands through the hair or rubbing along the skin. In an alternate embodiment, the user may place the film directly on wet hair or skin and distribute the film as desired through their hair or on the skin. In another embodiment, the film can be placed directly in the hands and then wetted and distributed throughout the hair or across the skin. Any other similar application method may be used.

If the cleansing film is used as a shaving film and contains surfactants, the film composition should not include harsh surfactants. For example, shaving films preferably do not contain sodium lauryl sulfate, a surfactant which can be very harsh and drying to skin.

A user of the personal care composition can apply the film to the hair or skin in a number of different ways. One method of application involves wetting the hands, placing the composition in the hands, distributing the film over the hands and then applying by passing the hands through the hair or rubbing along the skin. In an alternative embodiment, the user can place the film directly on wet hair or skin and distribute the film as desired through the hair or onto the skin. In another embodiment, the film can be placed directly in the hands and then wetted and distributed throughout the hair or across the skin. Any other similar application method can be used.

In another method of application of personal cleansing films according to the present invention, the personal cleansing film can be applied to the hair or skin by adding another product to a personal cleansing film to dissolve or disperse the personal cleansing film, and then applied together to the skin or hair. For instance, in one embodiment, a quantity of body wash can be added to a film in the hand and mixed in the hand to dissolve or disperse the film so that the mixture can be then applied to the body together. In another embodiment, a shave cream is dispensed onto the film in the hand, mixed by rubbing with the hands and then applied to the shaving area.

In another method of application of personal cleansing films according to the present invention, the personal cleansing film can be dissolved or dispersed in another application type in order to add to or increase the cleansing or lubricity properties of the application form. For instance, in one embodiment the personal cleansing film can be added to an existing body wash or shampoo to increase the efficacy of the body wash or change the aesthetic properties.

### EXAMPLES

The following examples are presented to further illustrate and explain the present invention and should not be taken as limiting in any regard.

The chemicals contained in the Examples are listed using their International Cosmetic Ingredient ('INCI') names or their tradenames. The INCI name of an ingredient comes from the International Cosmetic Ingredient Dictionary and Handbook, tenth ed.; T. E. Gottschalck and G. N. McEwen, Jr., Ph.D., J.D.; The Cosmetic, Toiletry, and Fragrance Association: Washington, D.C., (2003). For reference, tradenames used in the following Examples are listed next to their INCI name in the following Table 1-

**TABLE 1 - Tradenames with corresponding INCI and/or technical name**

| **Tradename** | **INCI Name** |
|---|---|
| MONATERIC 805 | Disodium cocoamphodiacetate |
| MONATERIC CAB-LC | Cocamidopropylbetaine |
| RHODAPEX ES-2 | Sodium lauryl ether sulfate |
| CRODASINIC LS-30 | Sodium Lauroyl Sarcosinate |
| AMISOFT CT12 | TEA-Cocoyl glutamate |
| STANDAPOL WAQ LC | Sodium lauryl sulfate |
| PLANTAREN W2000 | Decyl Glucoside |
| N-LITE LP | Food Starch-Modified |
| DOW CORNING 193 SURFACTANT | PEG-12 Dimethicone |
| DOW CORNING 2-8177 EMULSION | Amodimethicone |
| DOW CORNING 1664 EMULSION | Dimethicone and Laureth-4 and Laureth-23 |
| ULTRASPERSE A | Waxy maize starch |
| CELQUAT LS50 | Polyquaternium-4/Hydroxypropyl Starch Copolymer |
| STRUCTURE XL | Hydroxypropyl starch phosphate |

### EXAMPLE 1 - Procedural

In the Examples below, the following procedures are used -

Solution/Dispersion Batching: Solutions/dispersions used in casting films were made by mixing the solution/dispersion ingredients in a blender until homogeneous.

Film Casting: Films were cast by drawing down the solution/dispersion using a Braive Laboratory Bar Coater or a BASF Lab Coater onto a polyester sheet that was adhered to a flat surface and dried at 75°F (24°C) and 50% relative humidity for at least 10 hours. Where GAP liquid coating measurements are listed, the Braive Laboratory Bar Coater was used, and where liquid coating thicknesses above 100 are used, the BASF Coater was used.

Lather Life Time: Lather life time is determined by rubbing a 37mm x 60mm film section between wet hands then placing the lather onto a patch of skin and measuring the time for the lather to collapse and no longer resembles or appears as lather.

Cleansing Utility: Hand cleansing utility was determined by first wetting one hand with warm tap water, applying a test film to the wet hand, wetting the other hand with warm tap water and then rubbing the two hands together. Utility is shown when this action is sufficient to activate the film resulting in the dissolution of the film into an aqueous solution and distribution of the liquid through the hand.

### EXAMPLE 2 ― Polymers forming cleansing films

**TABLE 2**

| **Ingredient** | **1(g)** | **2 (g)** | **3(g)** | **4(g)** | **5(g)** |
|---|---|---|---|---|---|
| Pre-gelatinized corn starch | 15 | 0 | 0 | 0 | 0 |
| Hydrophobically modified waxy maize starch | 0 | 15 | 0 | 0 | 0 |
| Pre-gelatinized waxy maize starch | 0 | 0 | 15 | 0 | 0 |
| Polyvinyl pyrrolidone (K-90) | 0 | 0 | 0 | 15 | 0 |
| Pullulan | 0 | 0 | 0 | 0 | 15 |
| Sodium lauryl ether sulfate (25% aqueous solution) | 12 | 12 | 12 | 12 | 12 |
| Glycerin | 2 | 2 | 2 | 2 | 2 |
| Water | 68 | 68 | 68 | 68 | 68 |

The above compositions were batched and cast into films with a film thickness setting of 60 GAP according to the procedures above. Of the above compositions -

Composition 1 did not form a continuous film. Instead, flakes approximately 1 mm x 1 mm were found on the polyester sheet.

Composition 2 did not form a continuous film. Instead, long flakes approximately 1 mm x 10 mm were found on the polyester backing.

Composition 3 could not be coated because it formed a non-spreading gel.

Composition 4 formed a very flexible and tacky film.

Composition 5 gave a continuous film with high clarity.

Films resulting from compositions 4 and 5 were cut into rectangles that were approximately 37 mm x 60 mm. Their cleansing utility was demonstrated according to the procedure above. Lather and feel consistent with typical liquid hand wash was achieved by films from both compositions. The results of this Example demonstrate that not all film-forming polymers are suitable for creating a personal cleansing film.

### EXAMPLE 3 ― Surfactant Concentration Effects

**TABLE 3**

| **Ingredient** | **1 (g)** | **2 (g)** | **3 (g)** | **4 (g)** | **5 (g)** | **6 (g)** |
|---|---|---|---|---|---|---|
| Pullulan | 14.625 | 13.5 | 12.375 | 11.25 | 10.125 | 7.875 |
| Sodium Lauryl Ether Sulfate (25% aqueous solution) | 15 | 20 | 25 | 30 | 35 | 45 |
| Glycerine | 1.625 | 1.5 | 1.375 | 1.25 | 1.125 | 0.875 |
| Water | 68.75 | 65 | 61.25 | 57.5 | 53.75 | 46.25 |

The above compositions were prepared by mixing the ingredients in a blender until homogeneous and then cast into films according to the procedures above. Liquid thickness was set at 60 GAP.

Samples 1-5 formed flexible and continuous films. Sample 6 did not form a continuous film. This Example illustrates that there is an upper limit or amount of surfactant that can be incorporated and cast into a functional film. Samples 1-3 gave films with good clarity, indicating that they are homogeneous. Samples 4 and 5 gave films with distinct white visual features on the order of 0.5 mm in size, indicating that the films are heterogeneous throughout the sample. Films formed from compositions 4 and 5 were cut into rectangles that were approximately 37 mm x 60 mm.

Hand cleansing utility was demonstrated with films 1-5 by the procedure outlined in Example 1 above. Lather and feel consistent with a typical liquid hand wash was achieved for films 1-5, but more lather and more slippery feel were noted as one increased the level of surfactant in the film.

This Example illustrates that there is an upper limit to the amount of surfactant incorporated into a film using pullulan as the water soluble polymer. The data also demonstrates that a film can be heterogeneous and still provide a cleansing benefit.

### EXAMPLE 4 - Pullulan-modified starch blends

**TABLE 4 -**

| **Ingredients** | **1 (g)** | **2 (g)** | **3 (g)** | **4 (g)** |
|---|---|---|---|---|
| Pullulan | 3.09375 | 6.1875 | 9.28125 | 12.375 |
| Hydrophobically modified waxy maize starch | 9.28125 | 6.1875 | 3.09375 | 0 |
| Sodium lauryl ether sulfate (25% solids aqueous solution) | 25 | 25 | 25 | 25 |
| Glycerine | 1.375 | 1.375 | 1.375 | 1.375 |
| Water | 61.25 | 61.25 | 61.25 | 61.25 |

The above film compositions were batched and cast into films with a coating setting of GAP 60 according to the procedures in Example 1 above. Films 1 and 2 were very brittle films. Films 3 and 4 were flexible and homogeneous. Films formed from compositions 3 and 4 were cut into rectangles that were approximately 37 mm x 60 mm. Hand cleansing could be affected with films 3 and 4. Lather and feel consistent with typical liquid hand wash is achieved for both films. However, Film 3 has a noticeably creamier and more luxurious hand feel than Film 4. This Example demonstrates that blends of film forming polymers can be employed, and that the personal cleansing experience can be modified based on the polymers or blends thereof that are present.

### EXAMPLE 5 - Surfactant Blends Used in Pullulan-Based Films

**TABLE 5 - Concentrations of surfactants used in film compositions**

| Tradename | INCI Name | % Active |
|---|---|---|
| MONATERIC 805 | Disodium cocoamphodiacetate | 42.2 |
| MONATERIC CAB-LC | Cocamidopropylbetaine | 34.5 |
| RHODAPEX ES-2 | Sodium lauryl ether sulfate | 32 |
| CRODASINIC LS-30 | Sodium Lauroyl Sarcosinate | 30 |
| AMISOFT CT12 | TEA-Cocoyl glutamate | 30 |
| STANDAPOL WAQ LC | Sodium lauryl sulfate | 29.8 |
| PLANTAREN W2000 | Decyl Glucoside | 50 |

**TABLE 6**

| Ingredient | 1 | 1B | 2 | 2B | 3 | 4 | 5 | 6 | 11 | 12B |
|---|---|---|---|---|---|---|---|---|---|---|
| Pullalan | 12.6 | 12.6 | 12.6 | 12.6 | 12.6 | 12.6 | 12.6 | 12.6 | 13.3 | 13.3 |
| Disodium Cocamphodiaceate (42.2% a.i.) | 4.26 | 4.26 | 0 | 0 | 0 | 0 | 0 | 0 | 0 | 0 |
| Cocamidopropyl betaine (34.5% a.i.) | 0 | 0 | 5.21 | 5.21 | 0 | 0 | 0 | 0 | 0 | 0 |
| Sodium lauryl ether sulfate (32% a.i.) | 16.6 | 0 | 16.6 | 0 | 16.6 | 16.6 | 16.6 | 16.6 | 16.6 | 0 |
| Sodium Lauroyl Sarcosinate (30% a.i.) | 0 | 0 | 0 | 0 | 6 | 0 | 0 | 0 | 0 | 0 |
| TEA Cocoyl glutamate (30% a.i.) | 0 | 0 | 0 | 0 | 0 | 6 | 0 | 0 | 0 | 0 |
| Sodium lauryl sulfate (29.8% a.i.) | 0 | 16.6 | 0 | 16.6 | 0 | 0 | 6 | 0 | 6 | 16.6 |
| Decyl Glucoside (50% a.i.) | 0 | 0 | 0 | 0 | 0 | 0 | 0 | 6 | 0 | 6 |
| Glycerine | 1.4 | 1.4 | 1.4 | 1.4 | 1.4 | 1.4 | 1.4 | 1.4 | 0.7 | 0.7 |
| Water | 65.14 | 65.14 | 64.19 | 64.19 | 63.4 | 63.4 | 63.4 | 63.4 | 63.4 | 63.4 |
| Total | 100 | 100 | 100 | 100 | 100 | 100 | 100 | 100 | 100 | 100 |

The above compositions (in weight %) were batched and cast into films with a film thickness setting of GAP 40 according to the procedures in Example 1 above. Films 1, 2, 4 and 5 had very much residue, while films 1B, 2B, 3, 6, 11 and 12B did not. All films are flexible and homogeneous. Only residue-free films were cut into rectangles measuring approximately 37mm x 60mm.

Hand cleansing could be affected with films 1B, 2B, 3, 6, 11 and 12B. Lather and feel consistent with typical liquid hand wash is achieved for all films. However, Film 1B, 2B and 12B have a noticeably creamier and more luxurious foam hand feel than Film 3, 6 and 11, which feel more neutral. This Example demonstrates that blends of film forming polymers can be employed and that the personal cleansing experience can be modified by the polymers that are present.

### EXAMPLE 6 - Optimization of non-residue forming films of EXAMPLE 5

**TABLE 7**

| Ingredients | **1(g)** | **2(g)** | **3(g)** | **4(g)** | **5(g)** | **6(g)** | **7(g)** | **8(g)** | **9(g)** | **10(g)** | **11(g)** | **12(g)** | **13(g)** |
|---|---|---|---|---|---|---|---|---|---|---|---|---|---|
| Pullulan | 18.9 | 20.0 | 21.0 | 18.9 | 20.0 | 18.9 | 18.9 | 18.9 | 20.0 | 20.0 | 20.0 | 20.0 | 20.0 |
| Cocamidopropyl betaine (34.5 %) | 4.3 | 4.3 | 4.3 | 0.0 | 0.0 | 0.0 | 7.8 | 0.0 | 0.0 | 0.0 | 0.0 | 0.0 | 0.0 |
| Sodium lauryl ether sulfate (32 %) | 0.0 | 0.0 | 0.0 | 18.8 | 18.8 | 19.7 | 0.0 | 19.7 | 19.7 | 0.0 | 19.7 | 19.7 | 0.0 |
| Sodium lauryl sulfate (29.8 %) | 20.1 | 20.1 | 20.1 | 5.0 | 5.0 | 0.0 | 21.1 | 0.0 | 9.1 | 21.1 | 9.1 | 9.1 | 21.1 |
| Glycoside (50 %) | 3.0 | 3.0 | 3.0 | 3.0 | 3.0 | 5.4 | 0.0 | 5.4 | 0.0 | 5.4 | 0.0 | 0.0 | 5.4 |
| Glycerine | 2.1 | 1.1 | 0.0 | 2.1 | 1.1 | 2.1 | 2.1 | 2.1 | 1.1 | 1.1 | 0.0 | 0.9 | 0.9 |
| Propylene glycol | 0.0 | 0.0 | 0.0 | 0.0 | 0.0 | 0.0 | 0.0 | 0.0 | 0.0 | 0.0 | 1.1 | 0.0 | 0.0 |
| Dow Corning 193 surfactant | 0.0 | 0.0 | 0.0 | 0.0 | 0.0 | 0.0 | 0.0 | 0.0 | 0.0 | 0.0 | 0.0 | 0.2 | 0.2 |
| Water | 51.5 | 51.5 | 51.5 | 52.2 | 52.2 | 53.9 | 50.0 | 53.9 | 50.3 | 52.5 | 50.3 | 50.3 | 52.5 |
| Sum | 100 | 100 | 100 | 100 | 100 | 100 | 100 | 100 | 100 | 100 | 100 | 100 | 100 |

The above compositions were prepared and cast into films with a thickness setting of 40 GAP according to the procedures above. Films 1, 4, 6, 8 and 10 had residue, while films 2, 3, 5, 7, 9, 11, 12 and 13 did not. All films were sturdy, flexible and homogeneous. Films were cut into rectangles that were approximately 37 mm x 60 mm. Hand cleansing could be affected with all films. Lather and feel consistent with typical liquid hand wash was achieved with all films.

Films with reduced glycerine content (e.g., films 1-5) lead to higher foam volume when applying the films with water to the hands. Additionally, the films by themselves had less of a residue feel when the level of glycerine was reduced. Comparison of films containing propylene glycol (film 11) versus films containing glycerin (film 9) shows that propylene glycol helps to reduce brittleness of the film.

The addition of PEG-12 dimethicone (Dow Coming 193 Surfactant) improves the overall film properties (Film 9 and 12; film 10 and 13) such as brittleness. This Example demonstrates that blends of film forming polymers can be employed, and that the personal cleansing experience can be modified by the polymers and surfactants present in the product.

### EXAMPLE 7 - Pullulan films with additives

**TABLE 8**

| Ingredients | Test Sample No. | | | | | | |
|---|---|---|---|---|---|---|---|
| | 3 | 11 | 13 | 11B | 13B | 13C | 13D |
| Pullulan | 21.0 | 19.9 | 19.9 | 21.0 | 20.9 | 20.9 | 20.7 |
| Cocamidopropyl betaine (34.5% solids aqueous solution) | 4.3 | 0.0 | 0.0 | 0.0 | 0.0 | 0.0 | 0.0 |
| Sodium lauryl ether sulfate (32 % solids aqueous solution) | 0.0 | 19.7 | 0.0 | 19.7 | 0.0 | 0.0 | 0.0 |
| Sodium lauryl sulfate (29.8 % solids aqueous solution) | 20.1 | 9.1 | 21.1 | 9.1 | 21.1 | 21.1 | 21.1 |
| Glycoside (50 % solids aqueous solution) | 3.0 | 0.0 | 5.4 | 0.0 | 5.4 | 5.4 | 5.4 |
| Glycerin | 0.0 | 0.0 | 0.9 | 0.0 | 0.0 | 0.0 | 0.0 |
| Propylene glycol | 0.0 | 1.0 | 0.0 | 0.0 | 0.0 | 0.0 | 0.0 |
| Dow Corning 193 surfactant | 0.0 | 0.0 | 0.1 | 0.0 | 0.2 | 0.0 | 0.0 |
| Dow Coming 2-8177 emulsion (14% solids aqueous solution) | 0.0 | 0.0 | 0.0 | 0.0 | 0.0 | 0.5 | 1.1 |
| Dow Corning 1664 emulsion (50% solids aqueous solution) | 0.0 | 0.0 | 0.0 | 0.0 | 0.0 | 0.2 | 0.3 |
| Water | 51.5 | 50.3 | 52.5 | 50.3 | 52.5 | 51.9 | 51.4 |
| Total weight percent | 100.0 | 100.0 | 100.0 | 100.0 | 100.0 | 100.0 | 100.0 |

The above compositions (in weight %) were prepared and cast into films in order to demonstrate different properties of the films. The liquid in Table 8 was coated to a thickness of 40 GAP. Composition 13 was also coated to a thickness of 20, 25 and 30 GAP.

Residue - None of the films made from the compositions of Table 8 had residue. All films were sturdy, flexible and homogeneous. Films were cut into rectangles that were approximately 37 mm x 60 mm and evaluated for performance. Lather and feel consistent with typical liquid hand wash was achieved with all films.

Texture - By using different silicone, a variation in film properties was observed. Adding Dow Coming 2-8114 and/or Dow Coming 1664 instead of Dow Coming 193 gave films that were smoother and had a more slippery feel.

Dissolution Rate - Varying the film thickness from GAP 40 to GAP 20 lowered the dissolution time of the film in the application process while still delivering an amount of surfactants that provided a desired amount of lather and foam volume required for a pleasant hand-wash sensation.

This Example demonstrates that blends of film forming polymers can be employed, and that the personal cleansing experience can be modified by the polymers, additives, and surfactants present in the film.

### EXAMPLE 8 ― Modified Food Starch Cleansing Films

**TABLE 9 - Starch-based film compositions**

| Ingredients | Sample ID / Weight (grams) | | | | | | |
|---|---|---|---|---|---|---|---|
| | A | B | C | D | E | F | G |
| N-LITE LP | 70 | 60 | 57.5 | 65 | 45 | 45 | 45 |
| Bubble surfactant base * | 18.0 | 34.8 | 29.0 | 32.2 | 27.5 | 27.5 | 27.5 |
| Glycerin | 9.7 | 11.2 | 17.6 | 6.9 | 0 | 0 | 0 |
| Water | 120 | 120 | 120 | 120 | 120 | 120 | 120 |

| | | | | | | | |
|---|---|---|---|---|---|---|---|
| * Sodium lauryl sulfate/diethanolamine lauryl sulfate/C₁₂-C₁₆ alcohols/water (53.8% solids) | | | | | | | |

Solutions A, B, C, and D in Table 9 above were batched, cast into films on a BASF lab coater and allowed to dry overnight. All solutions formed acceptable personal cleansing films that exhibited adequate durability, lather, and cleansing performance when evaluated.

Solutions E, F, and G were coated using the same BASF lab coater but did not form films at a film coating setting of 500, 650 and 800.

**TABLE 9B - Starch-based film compositions**

| Ingredients | Sample ID / Weight (grams) | | | |
|---|---|---|---|---|
| | H | I | J | K |
| N-LITE LP | 55 | 50 | 72.5 | 70 |
| Bubble surfactant base | 22.9 | 33.5 | 23.1 | 29.8 |
| Glycerin | 24.6 | 27.0 | 4.7 | 3.2 |
| Water | 120 | 120 | 120 | 120 |

Films H, I, J, and K were batched but formed films that were too brittle when coated on a BASF lab coater with thickness setting of 535.

### EXAMPLE 9 - Preparation of Cleansing Films from Commercial Shampoo

**TABLE 10**

| Ingredients | E (grams) | F (grams) | G (grams) | H (grams) |
|---|---|---|---|---|
| N-LITE LP | 65 | 60 | 67.5 | 70 |
| Herbal Essence® shampoo | 86.4 | 93.5 | 83.2 | 80.2 |
| Glycerin | 6.9 | 11.23 | 5 | 3.2 |
| Water | 120 | 120 | 120 | 120 |

The above compositions E, F, G and H were made to demonstrate the ability to make commercial shampoos into personal cleansing films. Each composition was batched and cast into a film on a BASF lab coater at thickness setting of 500. Composition E formed a film which had some residue. To prevent blocking from the small amount of residue on the film, the film was coated with DRY-FLO PC. This coating prevented blocking.

Composition E formed acceptable personal cleansing films that exhibited adequate durability, lather, and cleansing performance when evaluated as outlined in Example 1 above. Compositions F, G, and H did not form acceptable films. Each composition when dried was either too brittle (Compositions G and H) or had too much residue and blocking (Composition F).

### EXAMPLE 10 - Shaving Film Preparations

**TABLE 11**

| **Ingredient** | **1** | **2** | **3** | **4** | **5** | **6** |
|---|---|---|---|---|---|---|
| Pullalan | 66.5 | 55 | 66.5 | 49.9 | 49.9 | 49.9 |
| CELQUAT LS50 | 0 | 0 | 0 | 16.6 | 0 | 0 |
| STRUCTURE XL | 0 | 0 | 0 | 0 | 16.6 | 0 |
| PVP K90 | 0 | 0 | 0 | 0 | 0 | 16.6 |
| Sodium laureth silfate | 20 | 27 | 0 | 20 | 20 | 20 |
| Sodium Lauryl Sulfate | 5 | 9 | 20 | 5 | 5 | 5 |
| Decyl gluconate | 5 | 9 | 0 | 5 | 5 | 5 |
| Cocamidopropyl betaine | 0 | 0 | 10 | 0 | 0 | 0 |
| glycerin | 3.5 | 0 | 0 | 3.5 | 3.5 | 3.5 |
| Dissolution time | medium | medium | medium | slow | fast | fast |
| Film strength | strong | weak | strong | strong | strong | weak |
| Lather life time | 1:16 | 1:20 | 2:50+ | 2:50+ | 1:19 | 2:00 |

The above compositions (weight %) were prepared and cast into films with a thickness setting of GAP 25. The liquids were centrifuged before being coated onto a polyester sheet used in the film casting procedure described above. The liquid was coated to a thickness of 25 GAP.

Samples were evaluated for film strength, dissolution time, and lather life time. Lather life time is a desirable attribute to control as some consumers use the removal of lather during shaving as a sign to know where they have already shaved.

Comparison of composition 1 to composition 2 shows that an increase in surfactant level and removal of plasticizer (glycerin) has little impact on lather but negatively impacts the film strength.

Comparison of 1 to 3 shows that changing the surfactant can increase the lather life time dramatically without changing the dissolution time or film strength.

Comparison of 1 to 4, 5 and 6 demonstrates that use of a single polymer versus blends of different film forming polymers influences the foam lather life time.

Although the present invention has been described and illustrated in detail, it is to be understood that the same is by way of illustration and example only, and is not to be taken as a limitation. The spirit and scope of the present invention are to be limited only by the terms of any claims presented hereafter.

## Claims

1. Personal care composition comprising:
at least one water soluble polymer in an amount of 45 to 82 weight %; and
at least one surfactant in an amount of about 18 to 55 weight %;
wherein the composition is a dry film, and weight % based on total weight of dry film.

2. The at least one water soluble polymer of claim 1 further comprising synthetic polymers, natural polymers, or combinations thereof.

3. The at least one water soluble polymer of claim 2 wherein the at least one water soluble polymer is at least one or more natural polymers comprising pullulan, starch, or combinations thereof.

4. The at least one water soluble polymer of claim 3 wherein the at least one water soluble polymer comprises a ratio of pullulan to starch in the amount of 100:0 to 75:25.

5. Personal care composition of claim 1 further comprising at least one plasticizer, and
the at least one water soluble polymer further comprising a blend of starch and pullulan.

6. Personal care composition of claim 5, wherein the at least one plasticizer is present in an amount greater than 15 percent based upon weight of starch in the composition.

7. Personal care composition of claim 6, where in the at least one plasticizer is present in an amount greater than 18 percent based upon weight of starch in the composition.

8. Personal care composition of claim 1 further comprising at least one plasticizer, and the at least one water soluble polymer further comprising starch, wherein the at least one plasticizer is present in an amount greater than 18 percent based upon the weight of the starch in the composition.

9. The at least one surfactant of claim 1 further comprising sodium lauryl sulfate, ammonium lauryl sulfate, sodium laureth sulfate, ammonium lauryl sulfate, cocamidopropyl betaine, lauramidopropyl betaine, disodium cocoamphodiacetate, sodium lauroyl sarcosinate, TEA-cocoyl glutamate, or decyl glucoside or combinations thereof.

10. Personal care composition of claim 1 further comprising at least one neutralizing agent present in an amount sufficient to neutralize the free acid groups of the water soluble polymer.

11. Personal care composition of claim 1 wherein the film is a cleansing film or a shaving film.

12. A method of cleansing skin and/or hair comprising:
wetting the film of claim 1 with water in an amount sufficient to create a lather, and
applying the lather to the skin and/or hair.

13. A method of shaving a substrate comprising:
wetting the film of claim 1 with water in an amount sufficient to create a lather, and
applying the lather to the substrate to be shaved.

14. A method of preventing dissolvable films from blocking comprising coating the film of claim 1 with a starch selected from the group consisting of unmodified and modified starch.

15. The method of claim 11 wherein the starch is hydrophobically modified.
